# EUROPÄISCHE PATENTSCHRIFT

(11) **EP 2 054 110 B1**
(45) Veröffentlichungstag und Bekanntmachung des Hinweises auf die Patenterteilung: **28.10.2020**
(21) Anmeldenummer: 07785084.0
(22) Anmeldetag: 10.08.2007
(51) Int. Cl.: A61M 5/20, A61M 5/31, A61M 5/50, A61M 5/24, A61M 5/315

(54) **INJEKTIONSVORRICHTUNG MIT MECHANIKVERRIEGELUNG**
INJECTION DEVICE COMPRISING A MECHANICAL LOCK
DISPOSITIF D'INJECTION COMPRENANT UN VERROU MÉCANIQUE

(30) Priorität: 14.08.2006 DE 102006038123
(43) Veröffentlichungstag der Anmeldung: 06.05.2009
(73) Patentinhaber: TecPharma Licensing AG, 3400 Burgdorf (CH)
(72) Erfinder: HIRSCHEL, Juerg, CH-5000 Aarau (CH); MOSER, Ulrich, CH-3412 Heimiswil (CH); SCHRUL, Christian, CH-3400 Burgdorf (CH); TSCHIRREN, Markus, CH-3422 Kirchberg (CH)
(86) Internationale Anmeldenummer: PCT/CH2007/000389
(87) Internationale Veröffentlichungsnummer: WO 2008/019514

(56) Entgegenhaltungen:
- EP-A- 0 554 995
- WO-A-00/62839
- WO-A-01/72361
- WO-A-02/092153
- WO-A-03/000317
- WO-A-2007/082400
- WO-A2-00/62839
- WO-A2-02/092153
- DE-A1-102004 055 298
- DE-C1- 19 821 934
- US-A1- 2005 222 540

## Beschreibung

Die Erfindung bezieht sich auf eine Injektionsvorrichtung zum Sichern einer Mechanik, insbesondere zum Sichern einer Einstellmechanik oder eines Einstellelements einer Injektionsvorrichtung gegen eine Betätigung, wie zum Beispiel eine Sicherung vor Verdrehung des Einstellelements, wobei die Betätigungs- oder Verdrehsicherung durch ein Einschieben einer Ampulle in die Injektionsvorrichtung aufgehoben werden kann.

In dem Dokument WO02/092153 A2 wird ein wiederverwendbares Injektionsgerät beschrieben, wobei das Injektionsgerät eine Mutter aufweist, welche mit einem Gehäuse verkeilt ist, damit die Mutter relativ zu dem Gehäuse axial bewegbar und drehfest ist.

Wenn eine Injektionsvorrichtung verwendet wird, in welche eine Ampulle vor dem Gebrauch der Injektionsvorrichtung eingesetzt wird, wie zum Beispiel eine unmittelbar vor dem Gebrauch einzusetzende und abzumischende 2-Kammer-Ampulle, kann es zu Problemen kommen, wenn ein Benutzer der Injektionsvorrichtung schon vor dem Einsetzen der Ampulle einen Einstell- oder Bedienvorgang durchführt.

Es ist eine Aufgabe der Erfindung eine Injektionsvorrichtung mit einer Mechanikverriegelung vorzuschlagen, welche die Sicherheit beim Bedienungsablauf einer Injektionsvorrichtung, in welche eine Ampulle eingesetzt werden soll, erhöht.

Die Aufgabe wird durch den Gegenstand des unabhängigen Anspruchs gelöst. Vorteilhafte Ausführungsformen sind in den abhängigen Ansprüchen definiert.

Eine erfindungsgemäße Injektionsvorrichtung weist ein Gehäuse und ein in dem Gehäuse gelagertes oder mit dem Gehäuse verbundenes oder gekoppeltes Bedienelement auf, wobei erfindungsgemäß das Bedienelement, wie zum Beispiel ein Einstell-, Druck- oder Drehknopf, so in dem Gehäuse gelagert oder mit dem Gehäuse gekoppelt oder verbunden ist, dass das Bedienelement von einer ersten Halteverbindung in einer ersten Position im Bezug auf das Gehäuse der Injektionsvorrichtung gehalten, also zum Beispiel gegen eine axiale Verschiebung gesichert, werden kann. Dabei ist die Halteverbindung so ausgebildet, dass diese während und bevorzugt nach dem Einbringen oder Einsetzen oder Einschieben einer Ampulle gelöst wird, so dass das Bedienelement nach dem Einbringen der Ampulle in eine zweite bevorzugt axial in proximale Richtung zur ersten Halteposition versetzte Halteposition verschoben wird und dort von einer zweiten Halteverbindung gehalten wird. Dabei wird vorzugsweise das Bedienelement durch das Einbringen oder Einschieben der Ampulle ebenfalls relativ zum Gehäuse der Injektionsvorrichtung verschoben, also zum Beispiel in proximale Richtung aus der Injektionsvorrichtung herausgeschoben. Jedoch kann die Injektionsvorrichtung auch so aufgebaut sein, dass eine Kupplung oder ein Koppelelement vorgesehen ist, welches durch das Einschieben der Ampulle verschoben wird, indem es zum Beispiel in Anlage mit einem proximalen Ampullenrand kommt, und so das Bedienelement freigibt. Dabei kann das Bedienelement selbst relativ zur Injektionsvorrichtung still stehen oder ebenfalls mit verschoben werden.

Vorzugsweise ist das Bedienelement oder Koppelelement so in der Injektionsvorrichtung gelagert, dass erst nach dem vollständigen Einsetzen oder Einschieben oder Eindrehen einer Ampulle, was mit dem Abmischen von zum Beispiel zwei in der Ampulle enthaltenen Substanzen einhergehen kann, aus der ersten Halteverbindung in die zweite Halteverbindung verschoben wird. Beispielsweise kann das Bedien- oder Koppelelement so in der Injektionsvorrichtung vorgesehen sein, dass eine einzubringende oder einzuschiebende Ampulle mit einem zum Beispiel vorher bekannten Maß oder Abmessungen erst auf den letzten Teil der Einschubstrecke mit dem Koppel- oder Bedienelement in Berührung kommt, so dass die Ampulle vor dieser letzten Wegstrecke in die Injektionsvorrichtung zum Beispiel eingeschraubt werden kann, ohne das Koppel- oder Bedienelement zu berühren bzw. zu verschieben und erst die nachfolgende Berührung der Ampulle mit dem Koppel- oder Bedienelement und die Verschiebung durch das dann vollständige Einschieben der Ampulle dazu führt, dass das Koppelelement die Mechanik zur Bedienung der Injektionsvorrichtung entriegelt oder dass das Bedienelement zum Beispiel durch Ausfahren aus dem Gehäuse der Injektionsvorrichtung für die Bedienung und zum Beispiel Einstellung durch einen Benutzer freigegeben wird.

Die erste und/oder zweite Halteverbindung können zum Beispiel durch eine Rastverbindung realisiert werden, wobei beispielsweise ein Rastring vorgesehen sein kann, welcher von einem Koppel- oder Bedienelement radial nach ihnen oder nach außen ragt und zusammen mit einer Ringnut der Injektionsvorrichtung oder des Gehäuses eine erste Halteverbindung bildet und einer weiteren Ringnut der Injektionsvorrichtung oder des Gehäuses die zweite Rastverbindung bildet. Ebenso kann das Halteelement auch durch andere mechanische Koppelungen realisiert werden, welche bevorzugt lösbar sind, wenn eine bestimmte Mindestkraft auf diese Kopplung einwirkt.

Die Mechaniksicherung kann zum Beispiel dadurch realisiert werden, dass eine Verdrehsicherung des Einstellelements zum Beispiel durch Führung von vorstehenden Nuten des Einstellelementes vorgesehen ist, so dass das Einstellelement in einer distalen Position gegen eine Verdrehung gesichert ist. Nach dem Eindrehen einer Ampulle wird das verdrehgesicherte Einstellelement so weit in proximale Richtung geschoben, dass die zur Verdrehsicherung dienenden Nuten aus dem diese Nuten haltenden und führenden Elementen zum Beispiel der Injektionsvorrichtung oder des Gehäuses herausgeschoben werden und somit eine Drehung und damit Betätigung des Einstellelementes möglich ist. Ebenso kann auch ein Koppelelement vorgesehen sein, welches eine Drehbewegung des Einstellelementes erst nach einer Verschiebung in proximale Richtung freigibt. Beispielsweise kann dieses Koppelelement ringförmig aufgebaut sein und nach innen und außen weisende Stege haben, welche in Nuten des Einstellelements und Nuten der Injektionsvorrichtung bzw. des Gehäuses eingreifen und so eine Verdrehung des Einstellelementes gegen das Gehäuse der Injektionsvorrichtung verhindern. Wird das Einstellelement durch eine in die Injektionsvorrichtung eingebrachte und zum Beispiel eingedrehte Ampulle zum Beispiel gegen die Kraft einer das Koppelelement in distale Richtung vorspannende Feder verschoben, bis die Stege des Koppelelementes aus den Nuten des Einstellelements und/oder aus den Nuten der Injektionsvorrichtung herausgeschoben werden, so wird die Kopplung der Einstellvorrichtung mit der Injektionsvorrichtung gelöst und die Injektionsvorrichtung kann nach dem bevorzugt vollständigen Einschieben der Ampulle in die Injektionsvorrichtung betätigt werden.

Die Erfindung wird nachfolgend anhand von Ausführungsbeispielen beschrieben. Es zeigen:
- Figur 1: eine Ausführungsform einer Injektionsvorrichtung in Draufsicht;
- Figur 2: eine perspektivische Querschnittansicht der Injektionsvorrichtung entlang des Schnittes A-A in Figur 1;
- Figur 3: eine Schnittbildansicht bei einer alternativen Ausführungsform entlang des Schnittes A-A von Figur 1 zusammen mit weiteren Schnitten B-B und C-C der Schnittbildansicht;
- Figur 4: die Injektionsvorrichtung der Figur 3 nach dem Abmischen des Pens und herausgeschobener Mechanik;
- Figur 5: die Injektionsvorrichtung gemäss Figur 4 nach Einstellen der Dosis und Spannen der Feder;
- Figur 6: die Injektionsvorrichtung gemäss Figur 5 nach Ausschütten der Dosis bei blockierter Mechanik und entspannter Feder;
- Figur 7: eine Querschnittansicht einer Injektionsvorrichtung ohne eingesetzte Ampulle;
- Figur 8: das Detail A in Figur 7 zur Veranschaulichung des Gewindeeingriffes der Führungshülse in die Gewindestange;
- Figur 9: das abgewickelte Gewinde der Führungshülse gemäss Figur 8 mit unterschiedlichen Anlageflanken zur Führung verschiedener Gewindestangen mit einem Aussengewinde unterschiedlicher Steigung;

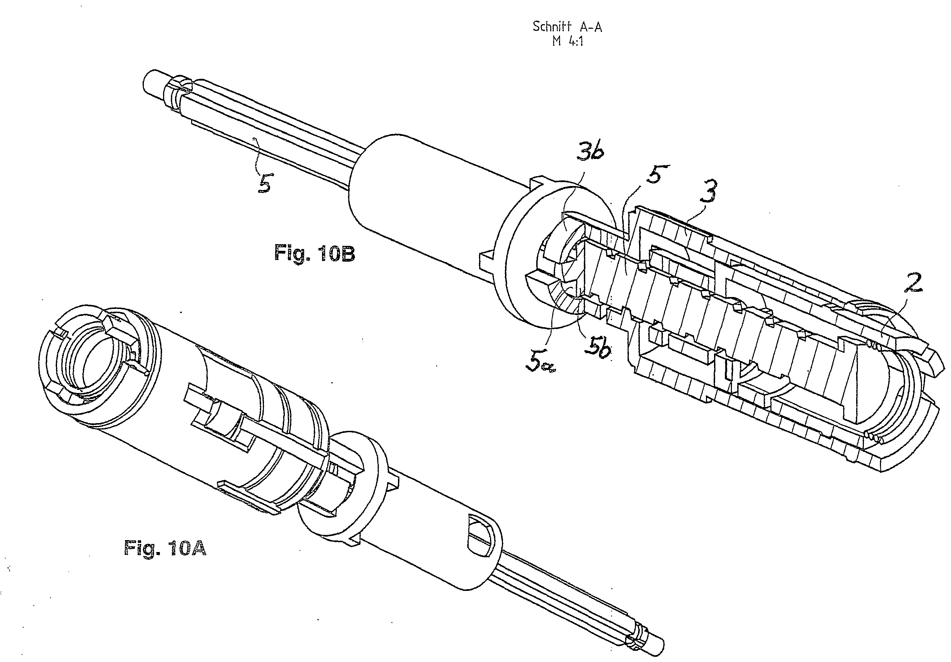

- Figur 10A: die Dosiermechanik der Injektionsvorrichtung in perspektivischer Ansicht;
- Figur 10B: die in Figur 10A gezeigte Dosiermechanik mit einer Querschnittsansicht des proximalen Teiles;
- Figuren 11A-11C: verschiedene Ausführungsformen einer Echtzeit- oder Restemengenanzeige;
- Figuren 12A-12B: eine Ausführungsform einer erfindungsgemässen Klauensicherung; und
- Figuren 13A-13G: eine Ausführungsform einer erfindungsgemässen Mechanikverriegelung

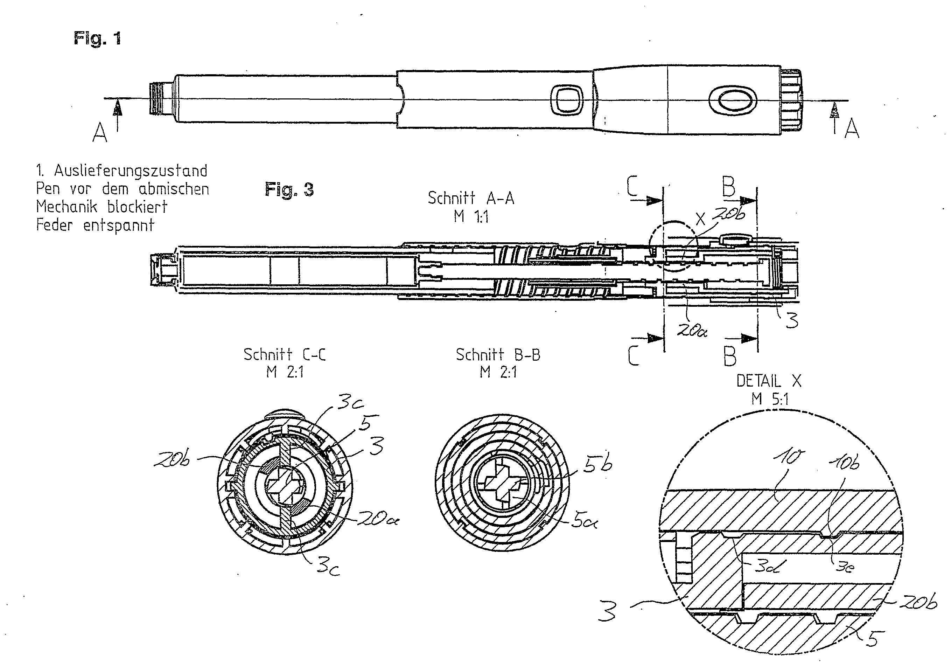

Fig. 1 zeigt einen fixed-dose-Pen, wobei die auszuschüttende Dosis am Dosierknopf 1 einstellbar ist.

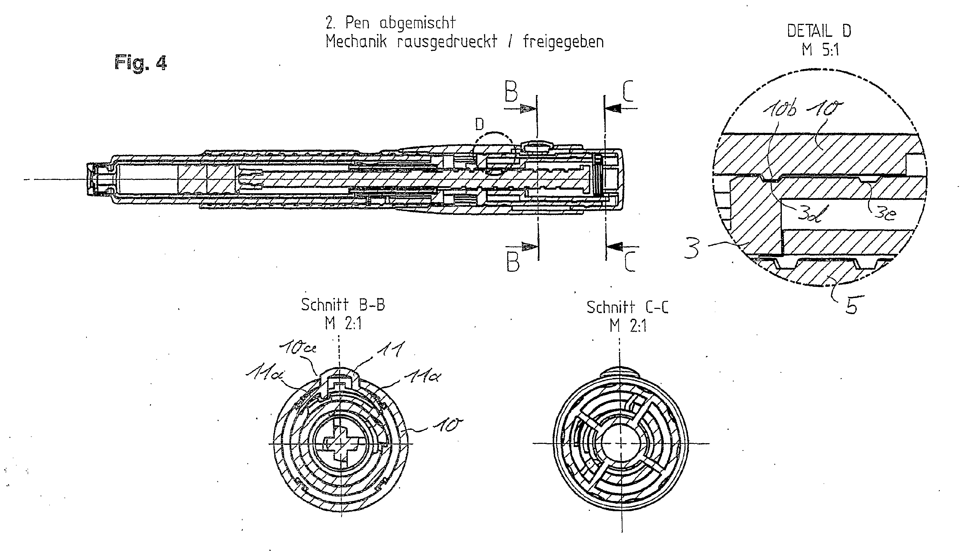

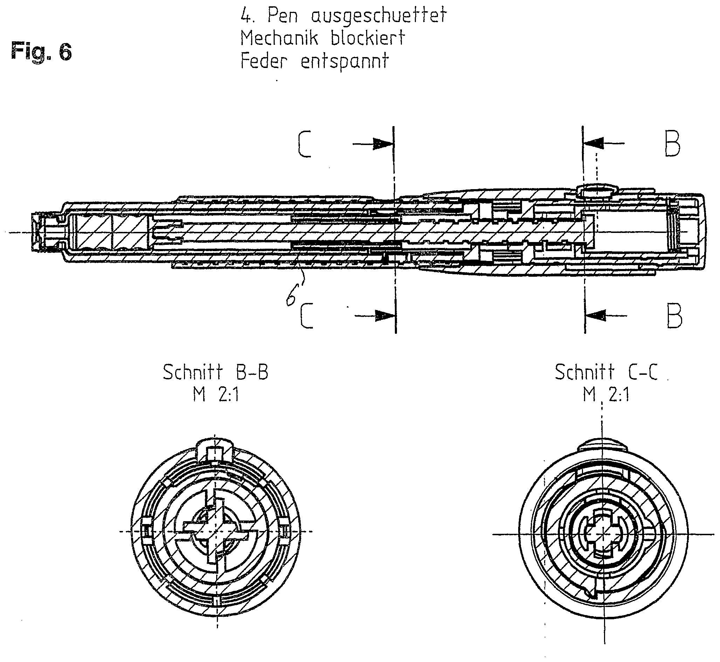

Der Pen weist eine Gewindestange 5 auf, welche wie in den Schnitten der Figuren 3 bis 6 gezeigt im. Querschnitt als Stern vier Zacken 5b aufweist, wodurch eine robuste und einfache Einstellung der fixed-dose möglich ist.

Es ist prinzipiell möglich, dass die Gewindestange 5 im Querschnitt sternförmig (Schweizer Kreuz-förmig) ausgebildet sein kann, jedoch kann der Stern auch mehr oder weniger Zacken Sb als das Schweizer Kreuz aufweisen.

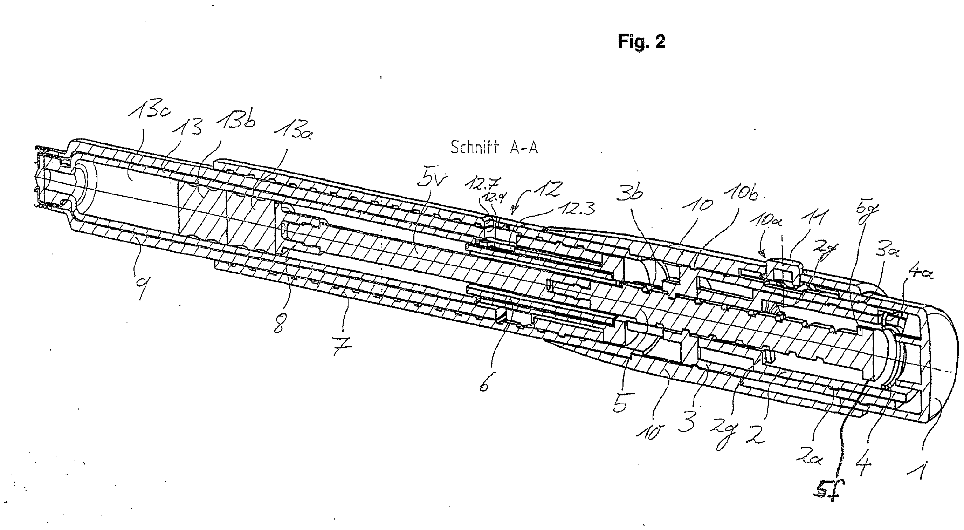

Fig. 2 zeigt den Pen gemäß Fig. 1 in perspektivischer Ansicht im Schnitt A-A.

Die Führungshülse 3 kann mit dem Gehäuse 10 verbunden sein und ist relativ zu dem Gehäuse 10 verdrehsicher gelagert. Innerhalb der Führungshülse 3 ist die Drehhülse 2 mittels einer Schnappwulst 2a drehbar, aber axial nicht verschiebbar, gelagert. Auf der Außenseite der Führungshülse 3 ist der Dosierknopf 1 ebenfalls mittels einer Schnappwulst 3a drehbar, aber nicht axial verschiebbar, gelagert. An der proximalen Seite der Drehhülse 2 und verbunden mit der Führungshülse 3 und der Drehhülse 2 befindet sich ein Federelement 4, in einer bevorzugten Ausführungsform als zwei- bis dreimal gewickelter Federdraht oder Federband, so dass, wenn die Drehhülse 2 relativ zur Führungshülse 3 gedreht wird, die Feder 4, welche zum Beispiel durch Aufbiegungen 4a an den entgegengesetzten Enden einmal mit der Führungshülse 3 und einmal mit der Drehhülse 2 verbunden ist, gespannt wird und so für eine Rückstellkraft gegen die Einstell-Drehbewegung sorgt.

Der Dosierknopf 1 wird bevorzugt axial nicht herausgezogen, sondern nur gedreht. Auf der Innenseite des Dosierknopfes 1 befinden sich in axiale Richtung weisend vier Stege, welche in entsprechende Nuten der Drehhülse 2 eingreifen und somit den Dosierknopf 1 mit der Drehhülse 2 so koppeln, dass eine Drehbewegung des Dosierknopfes 1 in eine Drehbewegung der Drehhülse 2 übertragen werden kann. Prinzipiell könnten Dosierknopf 1 und Drehhülse 2 auch einstückig oder als ein Element ausgebildet sein.

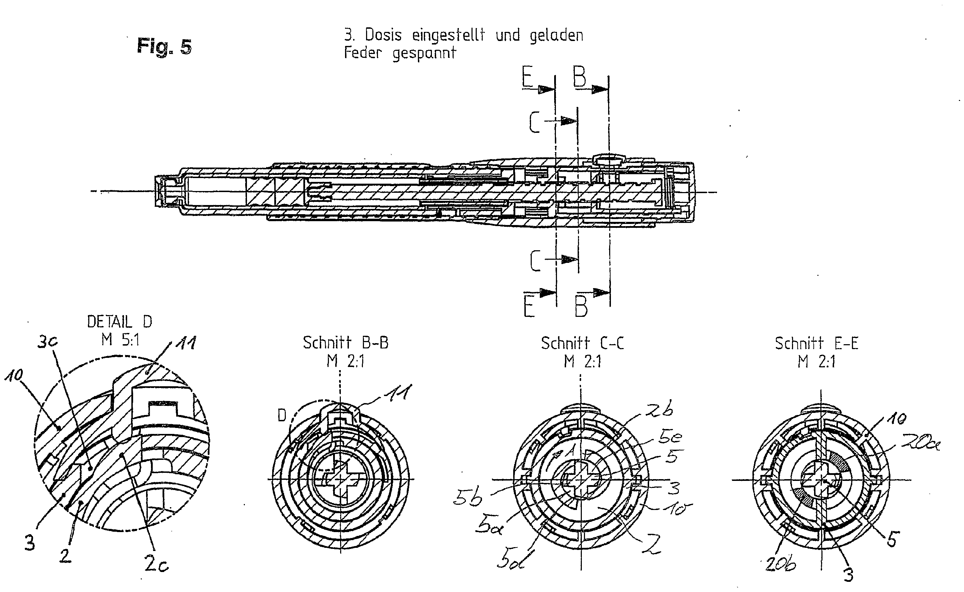

Die Drehhülse 2 weist radial nach innen vorgespannte Schnappelemente 2b, im Ausführungsbeispiel der Fig. 5 im Schnitt C-C gezeigt zwei gegenüberliegende radial nach innen vorgespannte Schnappelemente 2b, auf. Diese Schnappelemente 2b greifen in die vier Nuten 5a der Gewindestange 5 während der Aufziehbewegung ein oder werden an den Zacken 5b vorbeigedreht. Während des Aufziehvorganges ist die Gewindestange 5 verdrehgesichert gelagert, indem sie von einem Schnappelement 3b der Führungshülse 3 gehalten wird. Die Drehhülse 2 weist ein radial nach außen vorgespanntes Schnappelement 2c auf, welches in ein in Figur 5 im Schnitt B-B gezeigtes Fenster oder eine Durchbrechung 3c der Führungshülse 3 gedreht wird, wenn der Aufziehvorgang beendet ist, und verrastet in diesem Fenster 3c so, dass ein Zurückdrehen der Drehhülse 2 relativ zur und innerhalb der Führungshülse 3 aufgrund der Kraft der durch den Einstellvorgang vorgespannten Feder 4 nicht möglich ist. Wird der über dem Fenster 3c der Führungshülse 3 liegende Auslöseknopf 11 des Pens gedrückt, wird das radial nach außen vorgespannte Schnappelement 2c der Drehhülse 2 aus dem Fenster 3c der Führungshülse 3 herausgedrückt und somit die Drehhülse 2 relativ zur Führungshülse 3 freigegeben, so dass diese aufgrund der Kraft der vorgespannten Torsionsfeder 4 um den Einstellweg zurückgedreht werden kann.

Auf der distalen Seite der Drehhülse 2 sind in Umfangsrichtung gegenüberliegend zwei in axialer Richtung vorstehende Anschläge, Stege oder Nocken 2a, 2b vorgesehen, welche eine maximale Drehung der Drehhülse 2 von ca. 110° ermöglichen, da diese Nocken dann in Anlage mit ebenfalls in radialer Richtung, allerdings in proximale Richtung gerichtete, Nocken 3c der Führungshülse 3 kommen.

Beim Zurückdrehen in der im Schnitt C-C der Fig. 5 durch den Pfeil A gezeigten Ausschüttrichtung rasten die radial nach innen vorgespannten Nocken 2b der Drehhülse 2 in den in axialer Richtung verlaufenden Nuten 5a der Gewindestange 5, so dass die Gewindestange 5 von der Rückdrehbewegung der Drehhülse 2 mitgenommen wird und sich somit durch ein Innengewinde 3d der Führungshülse 3 geführt in distale Richtung in den Pen hineinschraubt. Dies bewirkt ein Vorschieben des oder der Stopfen 13a, 13b der Ampulle 13 durch den auf der distalen Seite der Gewindestange 5 oder eines damit verbundenen Verlängerungselements 5v vorgesehenen Stempel 8 in die Ampulle 13 hinein und somit eine Ausschüttung der in der Ampulle 13 enthaltenen Substanz 13c.

Nach erfolgter Ausschüttung kann der Pen durch Drehung des Dosierknopfes 1 wieder neu aufgezogen werden und die gleiche Dosis anschließend ausgeschüttet werden.

Die Gewindestange 5 kann im Spritzgussverfahren mit zwei Formhälften einfach hergestellt werden, wenn die Gewindestange 5 im Querschnitt ein einfaches Kreuz bildet. In axialer Richtung der Gewindestange 5 können die Nuten 5a, in welche der radial nach innen vorgespannte Schnapper 2b der Führungshülse 2 eingreift, durchgängig ausgebildet sein und unterbrechen so das Gewinde 5c auf der Außenseite der Gewindestange 5. Somit können hohe oder steile Flanken für den Schnapper 2b erzielt werden.

Die äußeren oder peripheren Bereiche der Gewindestange 5 weisen eine Schräge 5d auf, so dass der radial nach innen vorgespannte Schnapper 2b der Drehhülse 2 leichter beim Aufziehen darüber hinweg gleiten kann. Hierdurch ergibt sich auch eine höhere Flanke 5e auf der Seite der in axialer Richtung durchlaufenden Nut 5a der Gewindestange 5, in welcher der radial nach innen vorgespannte Schnapper 2b der Drehhülse 2 eingreift, wodurch ein Zurückdrehen der Drehhülse 2 relativ zur Gewindestange 5 sicher verhindert werden kann.

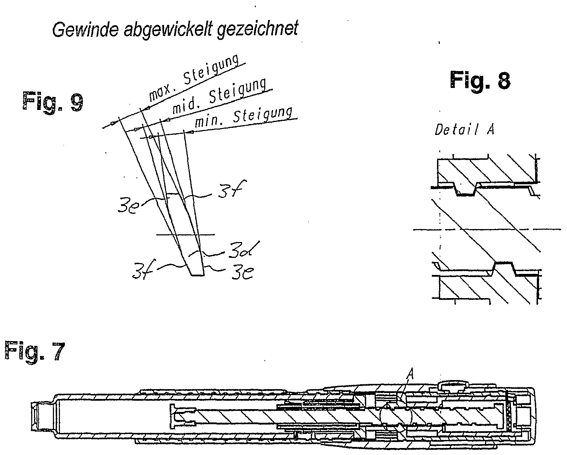

Prinzipiell ist es bei einer solchen Ausgestaltung der Gewindestange 5 relativ einfach möglich, die Gewindesteigung im Fertigungsprozess zu verändern, so dass bei gleicher Einstelldrehbewegung von zum Beispiel 110° unterschiedliche abzugebende Dosismengen in Abhängigkeit von der jeweils konkret vorliegenden Gewindesteigung realisiert werden können. Hierbei kann entweder · das Innengewinde 3d der Führungshülse 3 korrespondierend zur geänderten Steigung des Außengewindes 5c der Gewindestange 5 geändert werden. Alternativ ist es auch möglich, dass das Innengewinde 3d der Führungshülse 3 so ausgebildet ist, dass verschiedene Steigungen des zu führenden Außengewindes 5c der Gewindestange 5 geführt werden können, und zwar vollständig in einem Bereich von einer minimalen durch das Innengewinde 3d vorgegebenen Steigung bis zu einer maximalen durch das Innengewinde 3d vorgegebenen Steigung. Figur 9 zeigt einen einzigen Gewindegang des Innengewindes 3d der Führungshülse 3 in aufgerollter Darstellung mit einer minimalen und einer maximalen Steigung, welche sich durch Anlagekanten 3e und 3f des Innengewindes 3d ergeben.

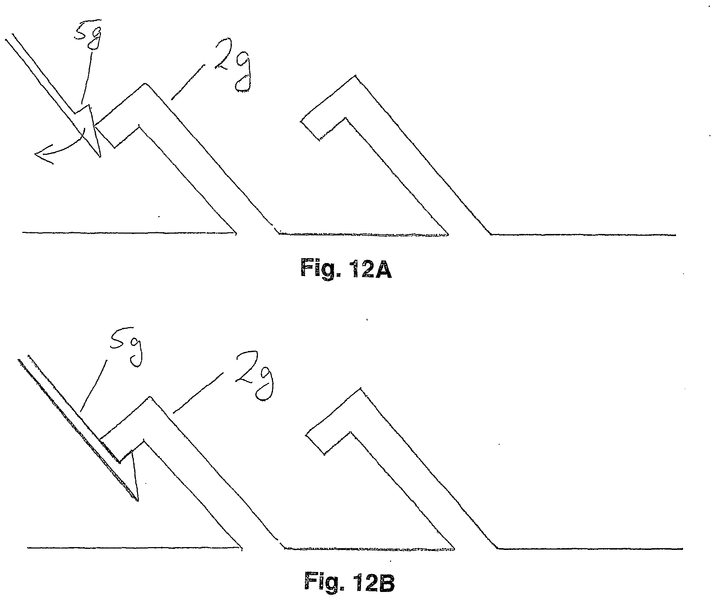

Um sicherzustellen, dass nach dem Ausschütten der letzten Dosis der Pen nicht mehr aufgezogen werden kann, ist an der Gewindestange 5 eine Klauensicherung vorgesehen. Diese weist an der proximalen Seite der Gewindestange 5 eine Erweiterung 5f auf, von welcher in axialer Richtung nach distal weisend zum Beispiel vier Stege 5g vorstehen, welche nach dem Ausschütten der letzten Dosis in entsprechende Gegenanschläge 2g der Drehhülse 2 einfahren oder in diese eingeschoben werden. Die Gewindestange 5 wird dabei axial so weit in die Drehhülse 2 hineinbewegt, dass die Klauen oder Stege 5g der Gewindestange 5 an den entsprechenden Gegenanschlägen 2g der Drehhülse 2 anliegen. Die Stege der Klauensicherung können in die entsprechenden Gegenanschläge 2g einfahren, indem sie beispielsweise bei einer Betätigung der Injektionsvorrichtung zumindest leicht oder teilweise mechanisch verformt oder komprimiert werden und sich zum Beispiel bei Erreichen der Endposition entspannen und in die beispielsweise als Ausnehmungen ausgebildeten Gegenanschläge einfahren. Die Klauen der Klauensicherung können auch federnd gelagert sein. Wird die Injektionsvorrichtung betätigt, gleiten die federnd gelagerten Klauen 5g, wie in Figur 12A gezeigt, an dem Gegenanschlag 2g entlang, indem sie in Pfeilrichtung ausgelenkt werden. Nach Abgabe der Dosis schnappen die Klauen 5g, wie in Figur 12B gezeigt, in den Gegenanschlag 2g ein, so dass die Injektionsvorrichtung nicht mehr aufgezogen werden kann. Unabhängig von der Ausgestaltung der Klauensicherung 5g und der Gegenanschläge 2g wird durch die erfindungsgemäße Ausgestaltung der Klauensicherung 5g und der Gegenanschläge 2g erreicht, dass nach dem Ausschütten der letzten Dosis der Pen nicht mehr aufgezogen werden kann, da die Gewindestange über den Formschluss zwischen Klauensicherung 5g und Gegenanschlägen 2g verdrehsicher gehalten wird. Ein weiteres Aufdosieren des Pens ist nicht mehr möglich, da die Gewindestange 5 verdrehsicher in der Führungshülse 3 gelagert ist und somit aufgrund der Klauenkupplung 2g, 5g ein Drehen des Dosierknopfes 1 bzw. der Drehhülse 2 verhindert wird.

Zum Einsetzen oder Eindrehen in die Injektionsvorrichtung kann eine Zweikammer-Ampulle 13 verwendet werden. Zum Abmischen wird die Ampulle 13 in den Pen hineingeschraubt, wodurch bei ausreichend weitem Hineinschrauben der Ampulle 13 in den Pen diese in Anlage an die Führungshülse 3 kommt und diese zusammen mit dem Dosierknopf 1 in proximale Richtung des Pens schiebt. Hierdurch wird der Dosierknopf 1 aus dem Pen herausgeschoben, was eine Einstellung oder ein Aufziehen des Pens überhaupt erst ermöglicht.

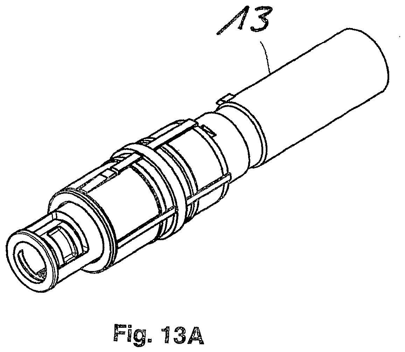

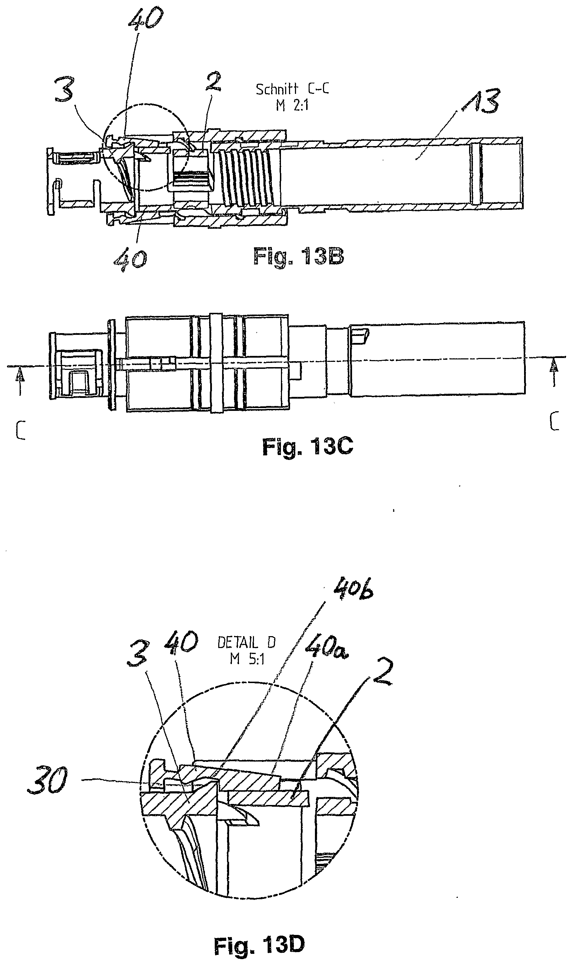

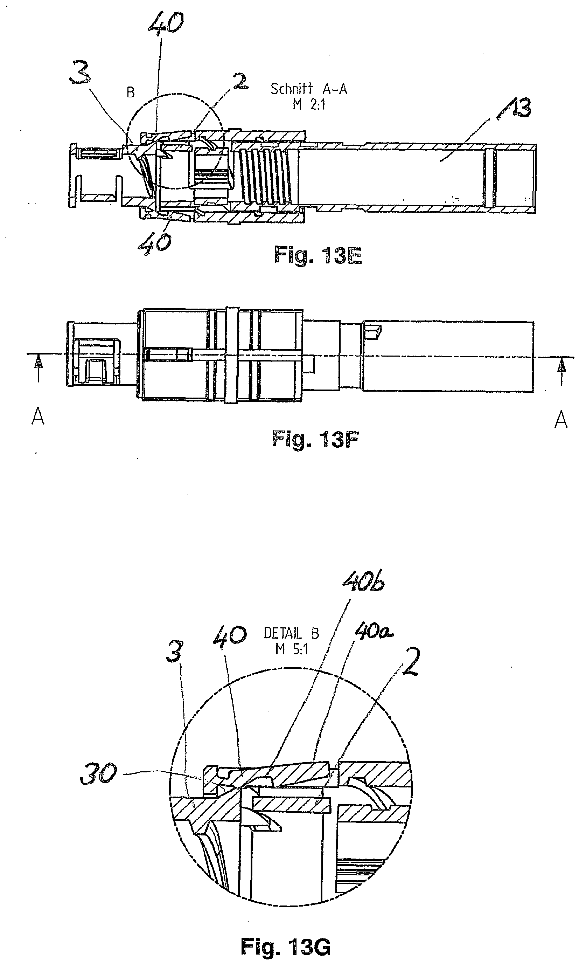

Wie in den Figuren 13B und 13C und der Detailansicht in Figur 13D gezeigt, ist ein Rastring 40 oder Sperrring vorgesehen, dessen zwei gabelförmigen Sperrklinken 40a in entsprechende Vertiefungen der Drehhülse 2 hineinragen und eine Drehung der Drehhülse 2 verhindern. Da die Injektionsvorrichtung durch ein Drehen der Drehhülse 2 geladen wird, wird das Aufziehen oder Laden der Injektionsvorrichtung durch das Eingreifen des Rastrings 40 in die Drehhülse 2 verhindert.

Zum Entsperren des Rastrings 40 und der Drehhülse 2 wird die Ampullenhülse, welche zum Abmischen der Zwei-Kammer-Ampulle in den Pen eingeschraubt wird, eingedreht. Auf den letzten Millimetern, wie den letzten 1 bis 3 mm, beispielsweise den letzen ca. 2 Millimetern, des Einschraubvorganges, wird der Rastring 40 durch die Ampullenhülse von der Sperrposition in eine entsperrte Position gebracht, in welcher der Rastring 40 nicht mehr mit der Drehhülse 2 verrastet ist. Ist die Ampulle 13 ausreichend weit eingedreht, werden die beiden Sperrklinken 40 des Rast- oder Sperrrings 40, wie in den Figuren 13E bis 13G gezeigt, hinausgedrückt, indem auf den Innenseiten der beiden gabelförmigen Sperrklinken 40a angeordnete schräge Flächen 40b oder Gleitflächen relativ zu an der Führungshülse 3 ausgebildeten schrägen Flächen 30 oder Gleitflächen entlang gleiten, so dass der Rastring 40 außer Eingriff mit der Drehhülse 2 gelangt und die Drehhülse 2 für Drehbewegungen, zum Beispiel für Dosis- oder Dosiereinstellungen, freigegeben wird.

Zusätzlich ist auf der Gewindestange 5 noch eine Anzeigenhülse 6 vorgesehen, die fest, d.h. drehfest und axial verschiebesicher, mit der Gewindestange 5 verbunden ist. Auf der Außenseite der Anzeigenhülse 6 sind in umlaufender Richtung die noch auszuschüttenden Dosismengen aufgezeichnet. Ein Sichtfenster 12 kann gebildet werden durch transparente Materialien oder Durchbrüche 12.3, 12.9, 12.7 in (von innen nach außen) Führungshülse 3, Ampullenhalter 9 und Gewindehülse 7.

Die Anzeigenhülse 6 ist bei abgemischter Ampulle 13 in diese eingeschoben (vorher nicht). Prinzipiell könnte die Anzeigenhülse 6 auch drehbar an dem hinteren Stopfen 13a angebracht sein, so dass die Anzeigenhülse 6 von der Mechanik des Pens zunächst entkoppelt ist und im Ampullenteil vorgesehen ist.

Da eine direkte Kopplung der Anzeigenhülse 6 mit der Gewindestange 5 besteht, kann die Anzeigenhülse 6 nicht verrutschen. Hierdurch kann es auch nicht zu einer Fehlanzeige kommen, selbst wenn der Pen zum Beispiel durch ein Herunterfallen einem starken Stoß ausgesetzt wird.

Die Führungshülse 3 dient auch als Sichtschutz, da das Fenster 12.3 in der Führungshülse 3 relativ zum Fenster 12.7 in der Gewindehülse 7 verschoben ist, bevor die Ampulle 13 eingeschraubt ist. Erst nach dem Einschrauben und Abmischen der Ampulle 13 wird das Fenster 12.3 der Führungshülse 3 in Deckung mit dem Fenster 12.7 der Gewindehülse 7 gebracht, so dass hierdurch die auf der Gewindestange 5 gelagerte Anzeigenhülse 6 sichtbar und ablesbar wird.

Der Auslöseknopf 11 wird durch ein Loch 10a in dem Gehäuse 10 positioniert und weist in Umlaufrichtung zwei Federarme 11a auf, welche den Auslöseknopf 11 radial nach außen weg von der Führungshülse 3 drücken. Die Federarme 11a beschreiben einen Radius, welcher kleiner als der Außenradius der Führungshülse 3 ist, so dass hierdurch die radial nach außen gerichtete Vorspannung des Auslöseknopfes 11 realisiert werden kann.

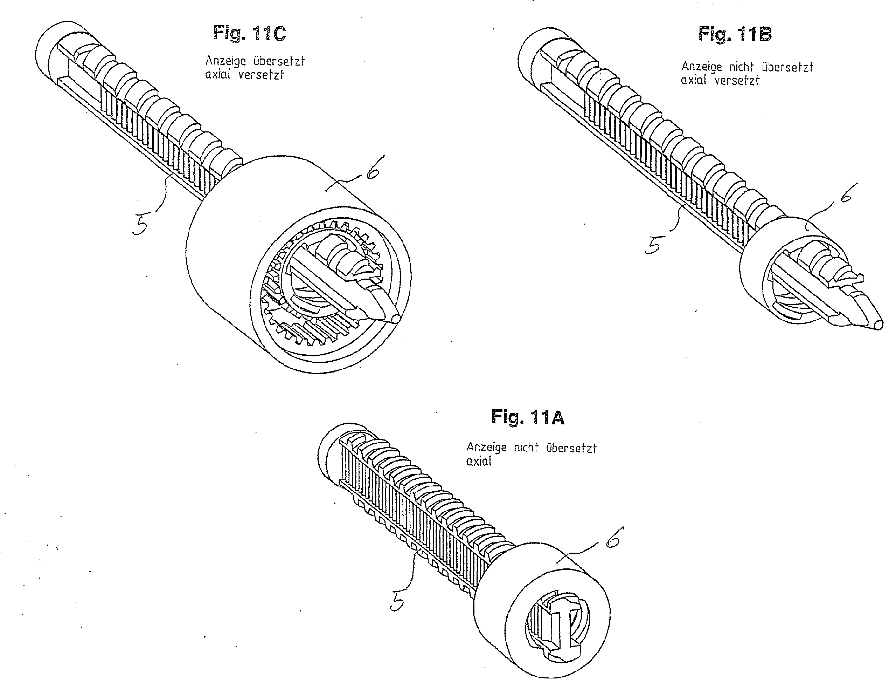

Die Anzeige 6 ist mit der Kolbenstange oder Gewindestange 5 in den in Fig. 11A und 11B gezeigten Ausführungsformen direkt gekoppelt und kann um diese ohne Selbsthemmung gedreht werden. Das Anzeigeelement 6 ist im Pen gegen axiale Verschiebung gesichert.

Die Kolbenstange 5 hat auf der Außenseite ein Gewinde oder Gewindeteilstück, in welches die Anzeige 6, oder bei Übersetzung der Anzeige 6 ein mit der Anzeige 6 gekoppeltes Übertragungselement, zum Beispiel ein Zahnrad oder wie in Fig. 11C gezeigt ein Zahnrad mit Innengewinde, eingreifen kann. Wird eine Übersetzung verwendet, so entsteht zwischen dem unmittelbar mit der Zahnstange gekoppelten Zahnrad und der Anzeige ein Zwischenraum, durch welchen zum Beispiel die Führungshülse 3 hindurchgeführt werden kann.

Wird anstatt der Zahnstange ein Drehmechanismus unter Verwendung einer Kolbenstange verwendet, so kann auch die Restmengenanzeige eingesetzt werden. Hierzu könnte das Restmengen-Anzeigelement 6 zum Beispiel auf der Kolbenstange 5 verdrehgesichert gelagert sein, so dass sich die Kolbenstange 5 während einer Ausschüttung durch die Restmengenanzeige 6 hindurchbewegt, die selbst axial verschiebegesichert in dem Pen gelagert ist.

Eine selbsthemmungsfreie Restmengenanzeige 6 kann durch eine geeignete Gewindesteigung realisiert werden, die materialabhängig ist und im Ausführungsbeispiel etwa 45° beträgt.

Vorzugsweise ist die Kopplung zwischen Restmengenanzeigeelement 6 und Zahnstange 5 so ausgelegt, dass bei vollständig hindurchgefahrener Zahnstange 5 das Restmengenanzeigeelement eine volle Drehung von 360° ausgeführt hat.

Bei einer Drehung von > 360° kann das Anzeigeelement 6 so ausgelegt sein, dass dieses mittels Außengewinde zusätzlich verschiebbar ist.

Denkbar wäre auch eine axial verschiebbare Restmengenanzeige 6, welche sich während des Hindurchfahrens der Zahnstange 5 in axiale Richtung des Pens bewegt, zum Beispiel durch einen Gewindeeingriff an der Außenseite der Restmengenanzeige 6 in ein Innengewinde im Gehäuse des Pens. Es kann zum Beispiel ein Pen mit konstanter voreingestellter Dosis verwendet werden, wobei die Restmengenanzeige zum Beispiel 14 maximal mögliche auszuschüttenden Einheiten anzeigt, die ausgehend von einem Anfangszustand auf 0 zurückgezählt werden.

## Patentansprüche

1. Injektionsvorrichtung mit einem Gehäuse (10) und einem als drehbaren Dosierknopf (1) oder drehbare Drehhülse (2) ausgeführten Bedienelement wobei der Dosierknopf (1) oder die Drehhülse (2) so in dem Gehäuse (10) gelagert ist, dass der Dosierknopf (1) oder die Drehhülse (2) in einer ersten Position gehalten wird und durch das Einbringen einer Ampulle (13) in eine axial zur ersten Position versetzte zweite Position verschoben wird, wobei eine Verdrehsicherung des Dosierknopfs (1) oder der Drehhülse (2) in der ersten Position vorgesehen ist, **dadurch gekennzeichnet, dass** ein Rastring (40) oder ein Sperrring (40) mit zwei gabelförmigen Sperrklinken (40a, 40b) als Verdrehsicherung vorgesehen ist, um in Vertiefungen des Dosierknopfs (1) oder der Drehhülse (2) einzugreifen, um eine Drehung des Dosierknopfs (1) oder der Drehhülse (2) zu verhindern.

2. Injektionsvorrichtung nach Anspruch 1, **dadurch gekennzeichnet, dass** in der zweiten Position der Rastring (40) oder der Sperring (40) unverrastet mit dem Dosierknopf (1) oder der Drehhülse (2) ist.

3. Injektionsvorrichtung nach Anspruch 1 oder 2, **dadurch gekennzeichnet, dass** an den Innenflächen der zwei gabelförmigen Sperrklinken (40a) schräge Flächen (40b) oder Gleitflächen (40b) angeordnet sind, welche an schräg ausgebildeten Flächen oder Gleitflächen einer Führungshülse entlang gleiten können, derart, dass der Rastring (40) oder der Sperring (40) ausser Eingriff mit dem Dosierknopf oder mit der Drehhülse (2) gelangt.

4. Injektionsvorrichtung nach einem der vorhergehenden Ansprüche, wobei durch das Einschieben der Ampulle (13) der Dosierknopf (1) oder die Drehhülse (2) aus dem Gehäuse (10) der Injektionsvorrichtung geschoben wird.

## Claims

1. An injection device comprising a housing (10) and an operating element in the form of a rotatable dosing knob (1) or rotatable rotary sleeve (2), wherein the dosing knob (1) or the rotary sleeve (2) is mounted in the housing (10) in such a way that the dosing knob (1) or the rotary sleeve (2) is held in a first position and is displaced by the introduction of an ampoule (13) into a second position displaced axially with respect to the first position, wherein there is provided a rotation-preventing means for the dosing knob (1) or the rotary sleeve (2) in the first position, **characterised in that** a latching ring (40) or a locking ring (40) with two fork-shaped locking pawls (40a, 40b) is provided as the rotation-preventing means in order to engage into recesses in the dosing knob (1) or the rotary sleeve (2) to prevent rotation of the dosing knob (1) or the rotary sleeve (2).

2. An injection device according to claim 1 **characterised in that** in the second position the latching ring (40) or the locking ring (40) is non-latched to the dosing knob (1) or the rotary sleeve (2).

3. An injection device according to claim 1 or claim 2 **characterised in that** arranged at the inside surfaces of the two fork-shaped locking pawls (40a) are inclined surfaces (40b) or sliding surfaces (40b) which can slide along inclined surfaces or sliding surfaces of a guide sleeve in such a way that the latching ring (40) or the locking ring (40) comes out of engagement with the dosing knob or the rotary sleeve (2).

4. An injection device according to one of the preceding claims wherein the dosing knob (1) or the rotary sleeve (2) is pushed out of the housing (10) of the injection device by insertion of the ampoule (13).

## Revendications

1. Dispositif d'injection avec un boîtier (10) et un élément de commande conçu comme un bouton de dosage rotatif (1) ou un manchon rotatif (2), dans lequel le bouton de dosage (1) ou le manchon rotatif (2) est logé dans le boîtier (10) de façon à ce que le bouton de dosage (1) ou le manchon rotatif (2) soit maintenu dans une première position et, grâce à l'insertion d'une ampoule (13), coulissée vers une deuxième position décalée axialement par rapport à la première position, dans lequel un dispositif anti-rotation du bouton de dosage (1) ou du manchon rotatif (2) dans la première position est prévu, **caractérisé en ce qu'**une bague d'encliquetage (40) ou une bague de blocage (40) avec deux cliquets de blocage en forme de fourche (40a, 40b), est prévu en tant que dispositif anti-rotation afin de s'emboîter dans des cavités du bouton de dosage (1) ou du manchon rotatif (2) afin d'empêcher une rotation du bouton de dosage (1) ou du manchon rotatif (2).

2. Dispositif d'injection selon la revendication 1, **caractérisé en ce que**, dans la deuxième position, la bague d'encliquetage (40) ou la bague de blocage (40) n'est pas encliquetée avec le bouton de dosage (1) ou le manchon rotatif (2).

3. Dispositif d'injection selon la revendication 1 ou 2, **caractérisé en ce que**, sur la surface interne des deux cliquets de blocage en forme de fourche (40a), sont disposées des surfaces inclinées (40b) ou des surfaces de glissement (40b), qui peuvent glisser le long de surfaces inclinées ou de surfaces de glissement d'un manchon de guidage, de façon à ce que la bague d'encliquetage (40) ou la bague de blocage (40) soit déboîtée du bouton de dosage ou du manchon rotatif (2).

4. Dispositif selon l'une des revendications précédentes, dans lequel l'insertion de l'ampoule (13) permet de pousser le bouton de dosage (1) ou le manchon rotatif (2) hors du boîtier (10) du dispositif d'injection.
